(19) Europäisches Patentamt
European Patent Office
Office européen des brevets

(11) Veröffentlichungsnummer : **0 064 953**
**B1**

(12) **EUROPÄISCHE PATENTSCHRIFT**

(45) Veröffentlichungstag der Patentschrift : **26.09.84**

(51) Int. Cl.³ : **C 07 C143/29**, C 09 B 23/14, C 11 D 3/42

(21) Anmeldenummer : **82810195.6**

(22) Anmeldetag : **10.05.82**

(54) **Stilbenverbindungen.**

(30) Priorität : **14.05.81 CH 3143/81**

(43) Veröffentlichungstag der Anmeldung : **17.11.82 Patentblatt 82/46**

(45) Bekanntmachung des Hinweises auf die Patenterteilung : **26.09.84 Patentblatt 84/39**

(84) Benannte Vertragsstaaten : **CH DE FR GB IT LI**

(56) Entgegenhaltungen :
**EP-A- 0 014 177**
**DE-A- 3 001 876**
**US-A- 3 984 399**

(73) Patentinhaber : **CIBA-GEIGY AG**
**Postfach**
**CH-4002 Basel (CH)**

(72) Erfinder : **Weber, Kurt, Dr.**
**Rennweg 98**
**CH-4052 Basel (CH)**

**EP 0 064 953 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft neue Stilbenverbindungen, ein Verfahren zu deren Herstellung sowie deren Verwendung als Zwischenprodukte zur Herstellung von optischen Aufhellern der Distyrylbiphenylreihe.

Aus der U.S. Patentschrift 3,984,399 ist bekannt, 4,4'-Distyrylbiphenyle aus 4,4'-Dialkoxyphosphorylmethyl-biphenylen und Benzaldehyden nach der Horner-Wittig-Synthese herzustellen. Die als Ausgangsstoffe verwendbaren Methan-phosphonsäureester werden aus 4,4'-Bis-chlormethylbiphenyl und dieses durch Chlormethylierung von Biphenyl erhalten. Dabei fallen jedoch Chlormethyl-biphenyle als Nebenprodukte an, die stark hautreizend sind.

Der vorliegenden Erfindung lag die Aufgabe zugrunde, Ausgangsstoffe zur Herstellung von optischen Aufhellern der 4,4'-Distyrylbiphenylreihe zu finden, die solche Nachteile nicht aufweisen.

Die erfindungsgemässen Stilbenverbindungen entsprechen der Formel

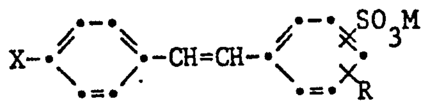

(1)

worin X Halogen, R Wasserstoff, $-SO_3M$, Halogen, $C_{1-4}$-Alkyl oder $C_{1-4}$-Alkoxy und M Wasserstoff oder ein salzbildendes Kation bedeuten.

Als Halogene X und R kommen Chlor, Brom und Fluor, besonders Chlor und Brom in Betracht.

Salzbildende Katione M sind z. B. Alkalimetall-, Ammonium- oder Aminsalzione. Unter Aminsalzionen sind solche der Formel $H^{\oplus}NR_1^0R_2^0R_3^0$ bevorzugt, in denen $R_1^0$, $R_2^0$ und $R_3^0$ unabhängig voneinander Wasserstoff, Alkyl, Alkenyl, Hydroxyalkyl, Cyanoalkyl, Halogenalkyl oder Phenylalkyl bedeuten oder worin $R_1^0$ und $R_2^0$ zusammen die Ergänzung zu einem 5-7-gliedrigen gesättigten Stickstoffheterocyclus darstellen, der noch zusätzlich ein Stickstoff- oder Sauerstoffatom als Ringglied enthalten kann, beispielsweise einen Piperidin-, Piperazin-, Pyrrolidin-, Imidazolin- oder Morpholinring, während $R_3^0$ für Wasserstoff steht.

Bevorzugte Stilbenverbindungen der Formel (1) sind solche der Formel

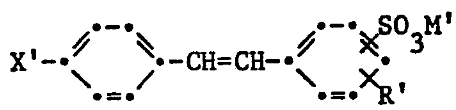

(2)

worin X' Chlor oder Brom, R' Wasserstoff, $-SO_3M'$, Chlor, Brom, $C_{1-4}$-Alkyl oder $C_{1-4}$-Alkoxy und M' ein Alkalimetall-, Ammonium- oder Aminsalzion bedeuten.

Besonders zu erwähnen sind die Stilbenverbindungen der Formel

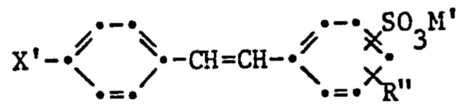

(3)

worin X' Chlor oder Brom, R'' Wasserstoff, Chlor, Methyl oder Methoxy und M' ein Alkalimetall-, Ammonium- oder Aminsalzion bedeuten.

Von praktischem Interesse sind die Stilbenverbindungen der Formeln

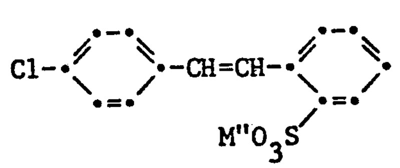

(4)

und

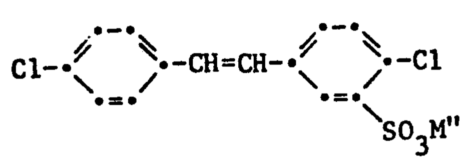

(5)

worin M'' Natrium oder Kalium bedeutet.

Die Stilbenverbindungen der Formel (1) können in an sich bekannter Weise hergestellt werden, indem man ein Mol einer Verbindung der Formel

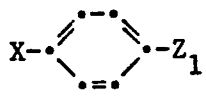

(6)

mit einem Mol einer Verbindung der Formel

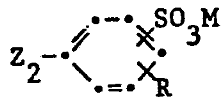

(7)

in Anwesenheit einer starken Base und eines polaren Lösungsmittels umsetzt, wobei eines der Symbole $Z_1$ und $Z_2$ eine HOC-Gruppe und das andere eine Gruppierung der Formel

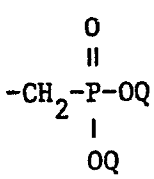 , 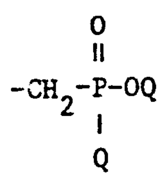 , 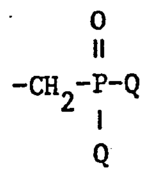 oder 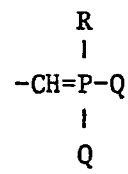

bedeuten, worin Q einen unsubstituierten oder substituierten Alkylrest, mit 1 bis 6 C-Atomen, einen Arylrest, vorzugsweise Phenylrest, einen Cycloalkylrest, vorzugsweise einen Cyclohexylrest oder einen Aralkylrest, vorzugsweise Benzylrest, darstellt.

Vorzugsweise stellt man die Verbindungen der Formel (1) her, indem man ein Mol einer Verbindung der Formel

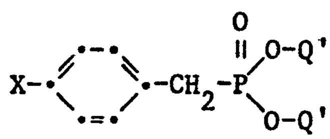

(8)

worin X die oben angegebene Bedeutung hat und Q'$C_{1-4}$-Alkyl bedeutet, in Gegenwart einer starken Base und eines polaren Lösungsmittels mit einem Mol einer Verbindung der Formel

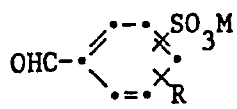

(9)

worin M und R die oben angegebene Bedeutung haben, umsetzt.

Als Lösungsmittel kommen z. B. Alkohole wie Methanol, Aethanol, Isopropanol, Butanol, Glykole, Glykoläther wie 2-Methoxyäthanol, Hexanole, Cyclohexanol und Cyclooctanol, ferner Aether wie Diisopropyläther, Tetrahydrofuran und Dioxan sowie Dimethylsulfoxyd, Formamid und N-Methylpyrrolidon in Betracht. Besonders geeignet sind polare organische Lösungsmittel wie Dimethylformamid und Dimethylsulfoxyd. Auch in wässriger Lösung lassen sich einige der Umsetzungen durchführen.

Die Temperatur, bei welcher die Umsetzung durchgeführt wird, kann in weiten Grenzen schwanken. Sie wird bestimmt :

α) durch die Beständigkeit des verwendeten Lösungsmittels gegenüber den Reaktionsteilnehmern, insbesondere gegenüber den stark basischen Alkaliverbindungen,

β) durch die Reaktivität der kondensationspartner und

γ) durch die Wirksamkeit der Kombination Lösungsmittel-Base als Kondensationsmittel.

In der Praxis kommen hiernach im allgemeinen Temperaturen zwischen etwa 10° und 100 °C in Betracht, insbesondere, wenn Dimethylformamid oder Dimethylsulfoxyd als Lösungsmittel verwendet werden. Der Temperatur-Vorzugsbereich liegt bei 20° bis 60 °C.

Als starke Basen kommen vor allem die Hydroxyde, Amide und Alkoholate (vorzugsweise solche primärer, 1 bis 4 Kohlenstoffatome enthaltender Alkohole) der Alkalimetalle in Betracht, wobei aus ökonomischen Gründen solche des Lithiums, Natriums und Kaliums von vorwiegendem Interesse sind.

Es können jedoch grundsätzlich und in besonderen Fällen auch Alkalisulfide und -carbonate, Arylalkaliverbindungen wie z. B. Phenyllithium oder stark basische Amine (einschliesslich Ammoniumbasen) z. B. Trialkylammoniumhydroxyde, mit Erfolg verwendet werden.

Die Stilbenverbindungen der Formel (1) können zur herstellung, nach an sich bekannter Weise, von bekannten optischen Aufhellern der 4,4'-Distyrylbiphenylreihe wie z. B. solche der U.S. Patentschrift 3,984,399, verwendet werden.

Die Herstellung erfolgt, indem man 2 Moläquivalente einer Stilbenverbindung der Formel (1) einer reduktiven Arylierung z. B. gemäss folgendem Schema

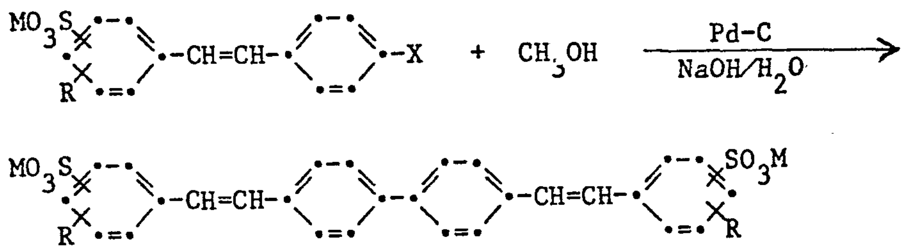


(2)

worin X, R und M die oben angegebene Bedeutung haben, unterwirft.

Die nachfolgenden Beispiele erläutern die Erfindung, ohne sie darauf zu beschränken. Prozente bedeuten, sofern nichts anderes angegeben ist, Gewichtsprozente.

Beispiel 1

530 g (4-Chlorphenyl)-methan-phosphonsäure-diäthylester (Gehalt : 98,8 %) und 442 g Benzaldehyd-2-sulfonsäure, Natriumsalz (Gehalt : 94 %) werden in 2 000 ml Dimethylformamid unter Rühren gelöst. Dann werden im Verlauf von 70 Minuten 396 g einer methanolischen Lösung von Natrium-methylat (Gehalt : 30 %) zugetropft, wobei die Temperatur auf 50 °C ansteigt. Dann wird 20 Stunden bei Raumtemperatur nachgerührt, das Reaktionsgemisch mit Salzsäure neutralisiert und unter Vakuum zur Trockne eingedampft. Der Rückstand wird bei 90 °C in 500 ml entsalztem Wasser gelöst, abkühlen gelassen, das auskristallisierte Produkt abgenutscht und unter Vakuum bei 110 °C getrocknet. Das so erhaltene Produkt wird zuerst aus 6 Liter wasserfreiem Alkohol und dann aus 250 ml entsalztem Wasser umkristallisiert und unter Vakuum bei 110 °C getrocknet. Man erhält so 387 g der Verbindung der Formel

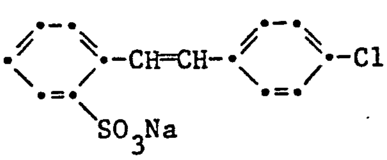

(100)

als weisse Nädelchen mit UV-Spektrum : $\lambda_{max.} = 302$ nm ; $\varepsilon = 29\,500$ ($H_2O$), welche noch 0,29 Mol Kristallwasser enthalten.

Der verwendete (4-Chlor-phenyl)-methan-phosphonsäure-diäthylester wurde auf folgende Weise erhalten :

495 g 4-Chlor-benzylchlorid und 1 531 g Triäthylphosphit werden unter Rühren langsam auf 150 °C erhitzt und während 7 Stunden bei dieser Temperatur gerührt. Dann wird das überschüssige Triäthylphosphit unter Wasserstrahlvakuum abdestilliert. Man erhält so 800 g eines blassgelben Oels mit einem Gehalt von 98,8 % an (4-Chlor-phenyl)-methan-phosphonsäure-diäthylester.

Beispiel 2

63,4 g der Verbindung (100) werden in 100 ml entsalztem Wasser bei 95 °C gelöst. Nach Zugabe von 23 g Kaliumchlorid und Abkühlen auf Raumtemperatur wird das auskristallisierte Produkt abgenutscht und mit einer 5 %-igen Kaliumchloridlösung gewaschen. Der feuchte Nutschkuchen wird bei 95 °C in 200 ml entsalztem Wasser gelöst und portionenweise soviel Kaliumchlorid zugegeben, bis eine leichte Trübung entsteht. Nach dem Abkühlen wird das auskristallisierte produkt abgenutscht, mit einer 5 %-igen Kaliumchloridlösung gewaschen, zuerst aus 200 ml entsalztem Wasser und dann aus einer Mischung von 50 ml entsalztem Wasser und 50 ml Aethanol umkristallisiert. Nach dem Trocknen erhält man 52 g der Verbindung der Formel

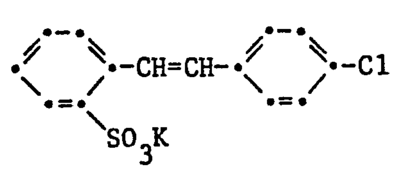

(200)

als weisses Kristallpulver mit UV-Spektrum : $\lambda_{max.}$ = 302 nm ($H_2O$), welches noch, 3,8 % Kristallwasser enthält.

Beispiel 3

52,5 g (4-Chlorphenyl)-methan-phosphonsäure-diäthylester (Gehalt : 98,8 %) und 51,2 g 4-Chlorbenzaldehyd-3-sulfonsäure, Natriumsalz (Gehalt 94,7 %) werden in 250 ml Dimethylformamid unter Rühren bei 40-45 °C gelöst. Dann werden im Verlauf von 20 Minuten 39,6 g einer methanolischen Lösung von Natrium-Methylat (Gehalt : 30 %) zugetropft, wobei die Temperatur bei 40-45 °C gehalten wird. Dann wird 3 Stunden bei 40-45 °C gerührt, das Reaktionsgemisch mit Salzsäure neutralisiert und unter Vakuum zur Trockne eingedampft. Der Rückstand wird bei 95 °C in 800 ml entsalztem Wasser gelöst, die trübe Lösung klärfiltriert, das Filtrat abkühlen gelassen, das auskristallisierte Produkt abgenutscht, aus 600 ml entsalztem Wasser nochmals umkristallisiert und unter Vakuum bei 120 °C getrocknet. Man erhält so 57 g der Verbindung der Formel

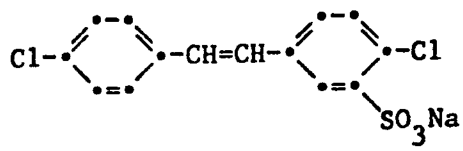

(301)

als weisse Kristalle.

UV-Spektrum $\lambda_{1\ max.}$ = 305 nm ; ε = 33'000, $\lambda_{2\ max.}$ = 320 nm ; ε = 35'000 (Dimethylformamid/$H_2O$ = 1 : 1)

Analog können unter Verwendung der entsprechenden Benzaldehydsulfonsäuren folgende Verbindungen erhalten werden.

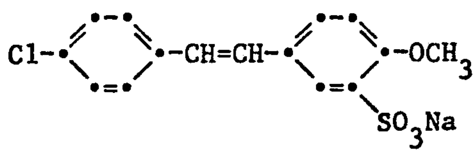

(302)

Weisse Kristalle.

UC-Spektrum : $\lambda_{1\ max.}$ = 304 nm ; ε = 31'000, $\lambda_{1\ max.}$ = 319 nm ; ε = 30'500 (Dimethylformamid/$H_2O$ = 1 : 1)

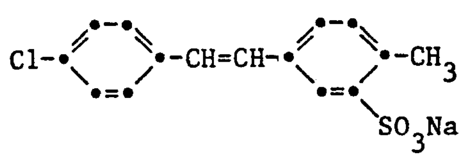

(303)

Weisse Kristalle.

UV-Spektrum : $\lambda_{1\ max.}$ = 305 nm ; ε = 32'000, $\lambda_{2\ max.}$ = 316 nm ; ε = 33'000 (Dimethylformamid/$H_2O$ = 1 : 1)

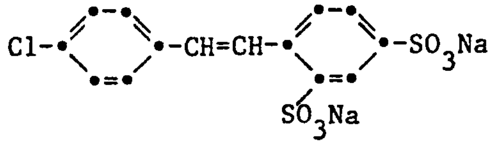

(304)

Weisse Kristalle.

UV-Spektrum : $\lambda_{max.}$ = 316 nm ; ε = 33'500 (Dimethylformamid/$H_2O$ = 1 : 1)

Beispiel 4

121 g (4-Bromphenyl)-methan-phosphonsäure-diäthylester (Gehalt : 98,5 %) und 88,5 g Benzaldehyd-2-Sulfonsäure Natriumsalz (Gehalt : 94,5 %) werden in 400 ml (Dimethylformamid unter Rühren gelöst.

Dann werden im Verlauf von 30 Minuten 79 g einer methanolischen Lösung von Natrium-methylat (Gehalt : 30 %) zugetropft, wobei die Temperatur auf 48 °C ansteigt. Dann wird 3 Stunden bei 40-50 °C nachgerührt, das Reaktionsgemisch mit Essigsäure neutralisiert und unter Vakuum zur Trockne eingedampft. Der Rückstand wird zweimal aus je 600 ml entsalztem Wasser umkristallisiert. Man erhält so 106 g der Verbindung der Formel

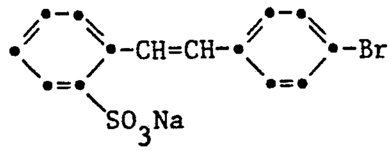

(400)

als weisse Kristalle.

UV-Spektrum : $\lambda_{1\ max.} = 305$ nm ; $\varepsilon = 30'500$, $\lambda_{2\ max.} = 315$ nm ; $\varepsilon = 30'000$ (Dimethylformamid/$H_2O$ = 1 : 1)

Der verwendete (4-Bromphenyl)-methan-phosphonsäure-diäthylester wurde auf folgende Weise erhalten :

100 g 4-Brom-benzylbromid und 300 ml Triäthylphosphat werden unter Rühren langsam auf 140 °C erhitzt und während 3 Stunden bei dieser Temperatur gerührt. Dann wird das überflüssige Triäthylphosphit unter Wasserstrahlvakuum abdestilliert. Man erhält so 121 g eines blassgelben Oels mit einem Gehalt von 98,5 % an (4-Bromphenyl)-methan-phosphonsäure-diäthylester.

Beispiel 5

16,1 g der Verbindung (100) werden in 70 ml entsalztem Wasser und 18,7 g einer 36 %-igen Lösung von Natronlauge in Wasser bei 75-80 °C gelöst. Nach Zugabe von 3 g 1 %-iger Palladiumkohle werden unter Rühren innert 3 Stunden 72 g Methanol zugetropft, wobei das Reaktionsgemisch unter Rückfluss siedet. Dann wird noch 21 Stunden unter Rückfluss gekocht, heiss von der Palladiumkohle abfiltriert, das Filtrat mit Salzsäure neutralisiert und zur Trockne eingedampft. Der Rückstand wird bei 90 °C in 100 ml Wasser gelöst und portionenweise mit Natriumchlorid versetzt, bis das Produkt auskristallisiert. Nach dem Abkühlen auf 5 °C wird das auskristallisierte Produkt abgenutscht, mit einer 10 %-igen Natriumchloridlösung gewaschen und unter Vakuum bei 120 °C getrocknet. Man erhält so 11,3 g eines hellgelben Kristallpulvers, welches 29,2 % der Verbindung der Formel

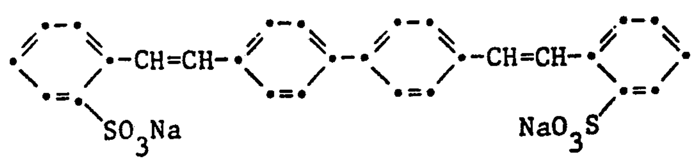

(500)

enthält.

**Ansprüche**

1. Stilbenverbindungen der Formel

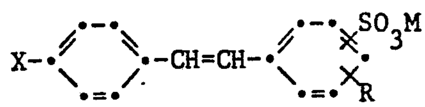

worin X Halogen, R Wasserstoff, —$SO_3M$, Halogen, $C_{1-4}$-Alkyl oder $C_{1-4}$-Alkoxy und M Wasserstoff oder ein salzbildendes Kation bedeuten.

2. Stilbenverbindungen gemäss Anspruch 1 der Formel

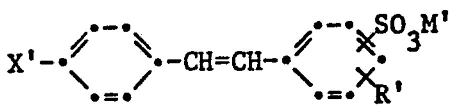

worin X′ Chlor oder Brom, R′ Wasserstoff, —$SO_3M'$, Chlor, Brom, $C_{1-4}$-Alkyl oder $C_{1-4}$-Alkoxy und M′ ein Alkalimetall-, Ammonium- oder Aminsalzion bedeuten.

3. Stilbenverbindungen gemäss Anspruch 2 der Formel

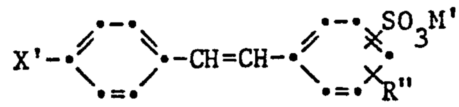

worin X' Chlor oder Brom, R'' Wasserstoff, Chlor, Methyl oder Methoxy und M' ein Alkalimetall-, Ammonium- oder Aminsalzion bedeuten.

4. Stilbenverbindungen gemäss Anspruch 3 der Formel

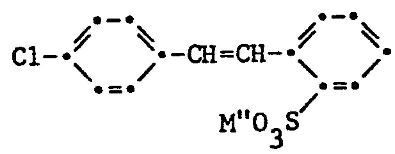

worin M'' Natrium oder Kalium bedeutet.

5. Stilbenverbindungen gemäss Anspruch 3 der Formel

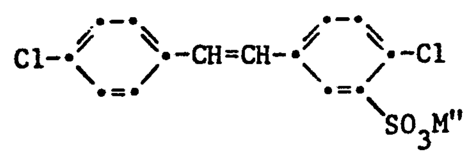

worin M'' Natrium oder Kalium bedeutet.

6. Verfahren zur Herstellung von Stilbenverbindungen der Formel

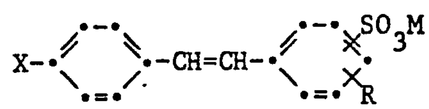

worin X Halogen, R Wasserstoff, —$SO_3M$, Halogen, $C_{1-4}$-Alkyl oder $C_{1-4}$-Alkoxy und M Wasserstoff oder ein salzbildendes Kation bedeuten, dadurch gekennzeichnet, dass man ein Mol einer Verbindung der Formel

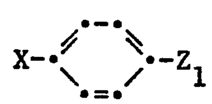

mit einem Mol einer Verbindung der Formel

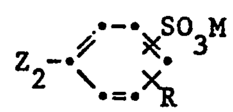

in Anwesenheit einer starken Base und eines polaren Lösungsmittels umsetzt, wobei eines der Symbole $Z_1$ und $Z_2$ eine HOC-Gruppe und das andere eine Gruppierung der Formel

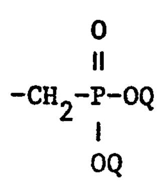 , 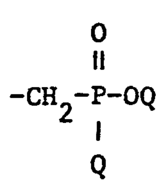 , 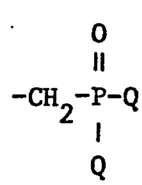 oder 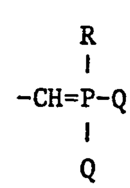

bedeuten, worin Q einen unsubstituierten oder substituierten Alkylrest mit 1 bis 6 C-Atomen, einen Arylrest, einen Cycloalkylrest oder einen Aralkylrest bedeutet.

7. Verfahren gemäss Anspruch 6, dadurch gekennzeichnet, dass man ein Mol einer Verbindung der Formel

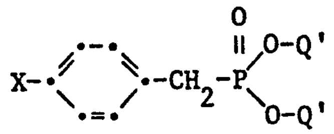

worin X die oben angegebene Bedeutung hat und Q′ $C_{1-4}$-Alkyl bedeutet, in Gegenwart einer starken Base und eines polaren Lösungsmittels mit einem Mol einer Verbindung der Formel

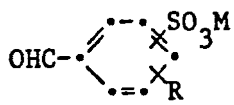

worin M und R die in Anspruch 6 angegebene Bedeutung haben, umsetzt.

8. Verwendung der gemäss Anspruch 6 erhältlichen Stilbenverbindungen als Ausgangsprodukte für die Herstellung von optischen Aufhellern der 4,4′-Distyrylbiphenylreihe.

## Claims

1. A stilbene compound of the formula

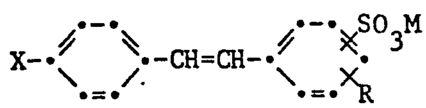

wherein X is halogen, R is hydrogen, $-SO_3M$, halogen, $C_1$-$C_4$-alkyl or $C_1$-$C_4$-alkoxy, and M is hydrogen or a saltforming cation.

2. A stilbene compound according to claim 1 of the formula

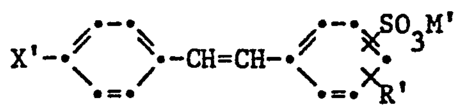

wherein X′ is chlorine or bromine, R′ is hydrogen, $-SO_3M'$, chlorine, bromine, $C_1$-$C_4$-alkyl or $C_1$-$C_4$-alkoxy, and M′ is an alkali metal ion, an ammonium ion or an amine salt ion.

3. A stilbene compound according to claim 2 of the formula

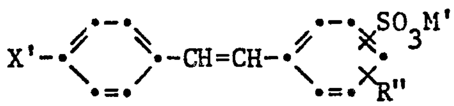

wherein X′ is chlorine or bromine, R″ is hydrogen, chlorine, methyl or methoxy, and M′ is an alkali metal ion, an ammonium ion or an amine salt ion.

4. A stilbene compound according to claim 3 of the formula

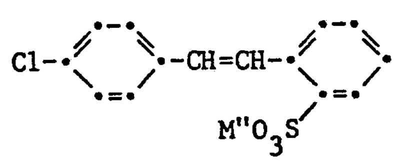

wherein M″ is sodium or potassium.

5. A stilbene compound according to claim 3 of the formula

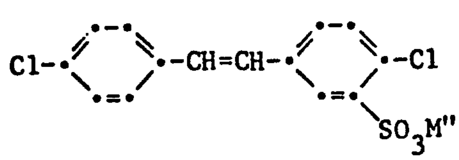

wherein M″ is sodium or potassium.

6. A process for the production of a stilbene compound of the formula

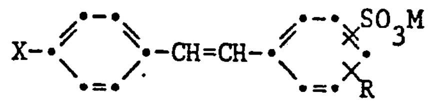

wherein X is halogen, R is hydrogen, —$SO_3M$, halogen, $C_1$-$C_4$-alkyl or $C_1$-$C_4$-alkoxy, and M is hydrogen or a salt-forming cation, which process comprises reacting one mole of a compound of the formula

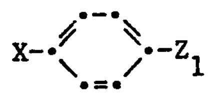

with one mole of a compound of the formula

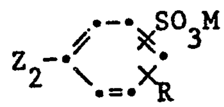

in the presence of a strong base and of a polar solvent, in which formulae one of the symbols $Z_1$ and $Z_2$ is a HOC group and the other is a group of the formula

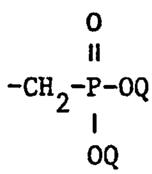 , 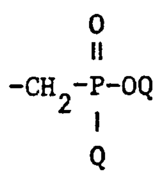 , 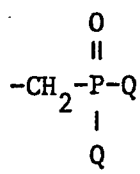 or 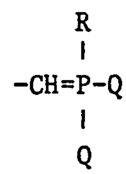

wherein Q is an unsubstituted or a substituted alkyl radical of 1 to 6 carbon atoms, an aryl radical, a cycloalkyl radical or an aralkyl radical.

7. A process according to claim 6, which comprises reacting one mole of a compound of the formula

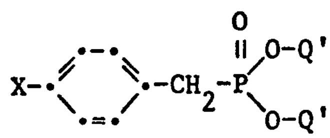

wherein X is as defined above and Q' is $C_1$-$C_4$-alkyl, in the presence of a strong base and of a polar solvent, with one mole of a compound of the formula

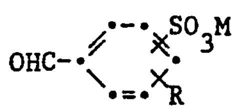

wherein M and R are as defined in claim 6.

8. Use of a stilbene compound obtainable according to claim 6 as starting material for obtaining fluorescent whitening agents of the 4,4'-distyrylbiphenyl series.

## Revendications

1. Dérivés de stilbène de formule

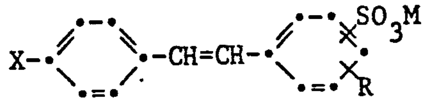

dans laquelle X est un atome d'halogène ; R est un atome d'hydrogène, —$SO_3M$, un atome d'halogène, un groupe $C_{1-4}$-alkyle ou $C_{1-4}$-alcoxy, et M est un atome d'hydrogène ou un cation salifiable.

2. Dérivés de stilbène selon la revendication 1, ayant la formule

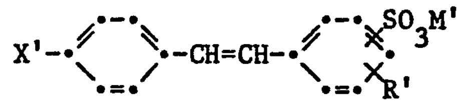

dans laquelle X' est un atome de chlore ou de brome ; R' est un atome d'hydrogène, $SO_3M'$, un atome de chlore, de brome, un groupe $C_{1-4}$-alkyle ou $C_{1-4}$-alcoxy, et M' est un ion de métal alcalin, d'ammonium ou de sel d'amine.

3. Dérivés de stilbène selon la revendication 2, ayant la formule

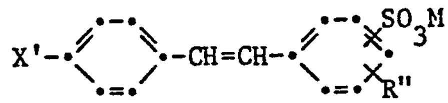

dans laquelle X' est un atome de chlore ou de brome ; R" est un atome d'hydrogène, de chlore, un groupe méthyle ou méthoxy, et M' est un ion de métal alcalin, d'ammonium ou de sel d'amine.

4. Dérivés de stilbène selon la revendication 3, ayant la formule

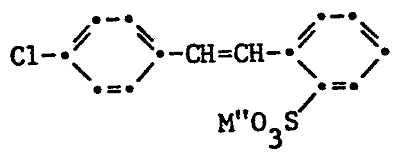

dans laquelle M" désigne le sodium ou le potassium.

5. Dérivés de stilbène selon la revendication 3, ayant la formule

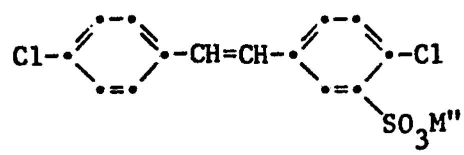

dans laquelle M" désigne le sodium ou le potassium.

6. Procédé pour la préparation de dérivés de stilbène ayant la formule

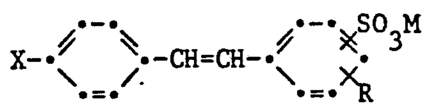

dans laquelle X est un atome d'halogène ; R est un atome d'hydrogène, $—SO_3M$, un atome d'halogène, un groupe $C_{1-4}$-alkyle ou $C_{1-4}$-alcoxy, et M est un atome d'hydrogène ou bien un cation salifiable, caractérisé par le fait qu'on fait réagir une mole d'un composé de formule

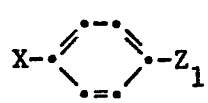

avec une mole d'un composé de formule

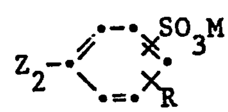

en présence d'une base forte et d'un solvant polaire, un des symboles $Z_1$ et $Z_2$ étant un groupe HOC et l'autre étant un groupe de formule

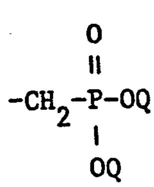 , 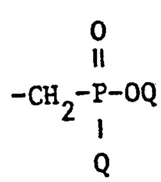 , 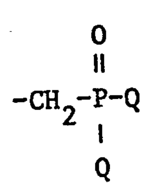 ou 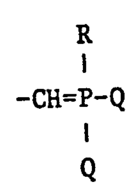

dans lesquelles, Q est un reste alkyle non substitué ou substitué ayant 1 à 6 atomes de carbone, un reste aryle, un reste cycloalkyle ou un reste aralkyle.

7. Procédé selon la revendication 6, caractérisé par le fait qu'on fait réagir une mole d'un composé de formule

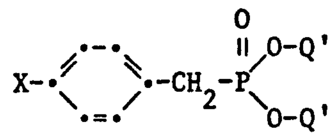

dans laquelle X a les significations données ci-dessus et Q' désigne un groupe $C_{1-4}$-alkyle en présence d'une base forte et d'un solvant polaire, avec une mole d'un composé de formule

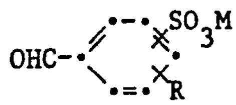

dans laquelle M et R ont les significations données dans la revendication 6.

8. Utilisation des dérivés de stilbène obtenus selon la revendication 6, comme produits de départ pour la préparation des azurants optiques de la famille des 4,4'-distyrylbiphényles.